# EUROPEAN PATENT SPECIFICATION

(11) **EP 3 706 639 B1**
(45) Date of publication and mention of the grant of the patent: **09.04.2025**
(21) Application number: 18808507.0
(22) Date of filing: 09.11.2018
(51) Int. Cl.: A61B 10/02, A61M 5/28, A61B 17/34, A61M 5/32, A61M 5/42, A61M 5/46, A61M 5/00

(54) **GUIDING DEVICE FOR INSERTING AND GUIDING A HARVESTING FAT TISSUE CANNULA AND RELATED STERILE KIT FOR HARVESTING ADIPOSE DERIVED STEM CELLS (ADSCS)**
FÜHRUNGSVORRICHTUNG ZUM EINFÜHREN UND FÜHREN EINER FETTGEWEBEENTNAHMEKANÜLE UND ENTSPRECHENDES STERILES KIT ZUR ENTNAHME VON STAMMZELLEN AUS FETTGEWEBE
DISPOSITIF DE GUIDAGE POUR INSÉRER ET GUIDER UNE CANULE DE TISSU GRAS DE RÉCOLTE ET KIT STÉRILE ASSOCIÉ POUR RÉCOLTER DES CELLULES SOUCHES DÉRIVÉES DU TISSU ADIPEUX (ADSC)

(30) Priority: 10.11.2017 IT 201700128569
(43) Date of publication of application: 16.09.2020
(73) Proprietor: Seffiline S.r.l., 40125 Bologna (IT)
(72) Inventor: Gennai, Alessandro, 40133 Bologna (IT)
(74) Representative: GCA S.R.L.
(86) International application number: PCT/IB2018/058823
(87) International publication number: WO 2019/092647

(56) References cited:
- WO-A1-2014/049713
- US-A- 4 713 053
- US-A- 5 241 969
- US-A1- 2011 313 345
- US-A1- 2012 123 386

## Description

### TECHNICAL FIELD

The invention relates to devices for harvesting adipose derived stem cells (ADSCs) and more in particular to a guiding device for inserting and guiding a harvesting tissue cannula in the subcutaneous adipose tissue of the patient, and to a related sterile kit to obtain adipose derived stem cells from the subcutaneous adipose tissue.

### BACKGROUND

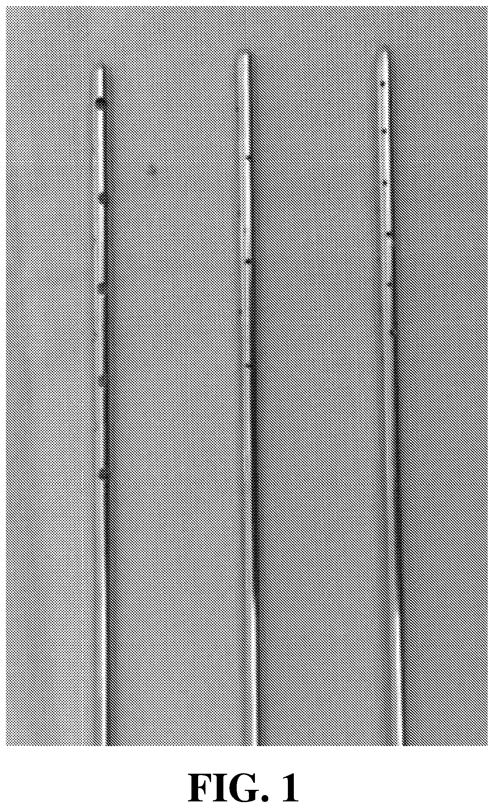

ADSCs are mesenchymal stem cells characterized by the ability to renew themselves through mitotic cell division and differentiate into a diverse range of specialized cell types. The ADSCs (Adipose Derived Stem Cells) are cells that abundantly exist in the SVF (Stromal Vascular Fraction) of the adipose tissue, and therefore, they are ideal as a cell source in the field of regenerative medicine. They may be obtained from fat depots, , and are harvested from adipose tissue through a simple, minimally invasive minimal-invasive harvesting procedure performed by a surgeon under general or local anesthesia. Harvesting procedure may be carried out for example according to the so-called The SEFFI and MicroSEFFI (or M-SEFFI) techniques, disclosed in the article *"*Superficial enhanced fluid fat injection (SEFFI and MicroSEFFI) in facial rejuvenation", by A. Gennai and F.P. Bernardini, CellR4 2017; 5 (1): e2239, and in the article *"*Skin Rejuvenation and Volume Enhancement with the Micro Superficial Enhanced Fluid Fat Injection (M-SEFFI) for Skin Aging of the Periocular and Perioral Regions", by A. Gennai et al.. According to these techniques, the harvesting procedure is performed using especially designed harvesting cannulas, that are depicted in figure 1. In order to get adipose tissue, these special cannulas are inserted under the skin and into the fat and are moved back and forth to collect adipose tissue in a syringe. The extraction of the mesenchymal stem cell ADSCs is usually done under local anesthesia and may involve the following process steps:
1. previous local anesthesia, through a little incision in the skin, inserting the cannula in the subcutaneous adipose tissue.
2. Harvesting procedure is performed collecting subcutaneous adipose tissue using the special harvesting cannula; the cannula is connected to a syringe of 10 ml. The adipose tissue with SVF and ADSCs is collecting in the syringe by creating a manual depression inside the syringe. The small side port holes cannula permit to collect small clusters of adipose tissue containing adipocytes, SVF and ADSCs.
3. After harvesting in the syringe a suitable compound of adipose tissue (an average of 5 ml), removing the cannula from the syringe, filling the syringe with Ringer lactate and closing the syringe with a cap.
4. Maintaining the syringe in the vertical position and in cold Ringer lactate in order to guarantee the cells viability. After few minutes there is the separation of tissue from washing liquid.
5. Removing washing liquid and leaving only the tissue with adipocytes, SVF with ADSCs in the syringe.
6. Proceeding with a gentle centrifuge (optional).
7. At the end of the centrifuge, removing oil and washing liquid again and leaving only tissue in the syringes containing adipocytes, SVF with ADSCs.
8. Connecting the syringe with the tissue to another syringe 10ml trough a special connector.
9. Transferring the tissue from a syringe to the other one, vigorously 20 times in order to obtain a very fluid injectable tissue containing mesenchymal stem cells (ADSCs).

The most critical step of the above procedure is the harvesting, that is performed by moving back and forth the cannula just under the skin of the patient for collecting adipose tissue. This operation is performed by a certified plastic surgeon because an improper use of the cannula, which is relatively long, may cause wounds to deeper tissues or inner organs of the patient. Moreover it is mandatory to harvest the adipose tissue in the very superficial layer above the skin: in this layer there is the highest concentration of SVF hence of ADSCs.

From US2011313345 a "Ultrasonic device for harvesting adipose tissue" is known. In this patent embodiments of methods and devices are described for removing adipose tissue from a surgical site or location in a patient's body. The embodiments include a device that is used for infiltration, ultrasound and aspiration of the surgical site. The device includes a cannula which serves to provide infiltration, conduct ultrasonic energy from an ultrasonic generating device, and also provide a conduit for aspiration to a fluid system used for infiltration and collection of fluids. Embodiments provide for a fixed amount of infiltration fluid to be injected into the surgical site in specific ratio with the amount of lipo-aspirate to be removed. The fixed amount of ultrasonic energy, both in amplitude and time, is delivered to the surgical site commensurate with the amount of infiltration and aspiration. The device also includes, in embodiments, a guide that limits the depth to which the cannula can be inserted into a patient.

From WO2014049713 a "Syringe storage container" is known.

The syringe storage container has annular scratches on the outer peripheral surface of a syringe so as to occur even when transporting plurality of medicine loading syringes that are hung and stored in receiving cylinders of a plate body. The syringe storage container stores a plurality of syringes that are each provided with a contacting part on a side face of a flange on an outer peripheral part of a cylindrical body part, the container comprising a container main body having a ledge on peripheral wall parts that are formed continuously around a bottom part of the lower end and extended toward the upper end; and a nest plate which is mounted on the ledge and has a plurality of aligned received cylinders penetrating therethrough that are spaced apart, the receiving cylinders being configured such that syringes are inserted and hung therefrom by the flanges being engaged therewith. Each receiving cylinder comprises a receiving cylinder main body which has an upper end with an engaging part, for the flange, formed thereon, and has an inner diameter that is larger than the outer diameter of the body part; and a protruding part which protrudes from the upper end to the upper end side and comes into contact with the contacting part when the flange is engaged with the engaging part. When a syringe is hung on a receiving cylinder, the protruding part comes into contact with the contacting part to regulate rotation of the syringe inside the receiving cylinder.

From US 4713053 a "Method and apparatus for performing suction lipectomy" are known.

A cannula is provided with a guide bar extending in spaced, parallel relationship to the cannula. Adjacent and overlying a hole formed in the cannula tip through which suction is applied to surgically aspirate fatty tissue is a guide surface adapted to contact and slide against the skin of a patient while the cannula tip is manually directed by the surgeon through the fatty tissue in reciprocating strokes. The guide surface, which may be a pair of wheels, maintains the tip at a constant depth within the tissue so that, upon completion of suction lipectomy, a desired amount of fatty tissue is surgically aspirated while leaving an even thickness layer of tissue intact. A new surgical procedure for performing suction lipectomies with the guided cannula of the invention is also disclosed. In accordance with a second embodiment of the invention, the guide bar includes an elevated portion spacer a greater distance from the cannula and a forward end of the guide bar having the guide surface. The elevated portion is manually engageable to permit two-handed reciprocating movement of the cannula by the surgeon and to allow the surgeon to contact the patient's skin beneath the elevated portion to assist the cannula in the surgical procedure.

From US 5241969 a "Controlled and safe fine needle aspiration device" is known.

A syringe holder which is uniquely designed for the diagnostic technique of fine needle aspiration used in the diagnosis of cancer and other pathological processes. The holder can be used repeatedly with a standard disposable syringe and a needle. A stabilizing ring with or without a detachable extended stabilizing ring is placed on the skin around the mass which is to be needled, and the needle is guided into the mass accurately as the syringe is held firmly by a syringe body holder which slides on guide bars. A vacuum is created by pulling the syringe plunger back with a plunger holder, and cells and tissue from the mass are sucked into the syringe as the needle is moved in an up-and-down direction by the hand holding a handlebar. The cells and tissue obtained, the vacuum is released. The needle is removed from the skin, and the sample is removed from the syringe.

It would be desirable a safety device for guiding the insertion and the movement of the cannula below the skin of the patient, that would make impossible any incorrect and potentially dangerous use thereof, thus that could be handled also by a less qualified person than a certified plastic surgeon, and moreover guide the operator to harvest tissue in the best layer of adipose tissue.

### SUMMARY

An excellent solution to at least part of the above-mentioned drawbacks is provided by a guiding device as defined in claim 1. The guiding device of this invention is shaped so as to hold a syringe equipped with the harvesting cannula and to allow to insert this cannula at the exact depth below the skin of a patient to attain the subcutaneous adipose tissue. The cannula may be moved back and forth in a driven manner immersed in the subcutaneous adipose tissue without any risk of wounding inner organs of the patient.

### BRIEF DESCRIPTION OF THE DRAWINGS

Figure 1 is a picture of three different harvesting cannulas.
Figure 2 is a top view of a prototype of a guiding device according to the invention with a central locking tooth for retaining a syringe for collecting ADSCs through the harvesting procedure from the subcutaneous adipose tissue of a patient.
Figure 3 is a side view of the guiding device of figure 2 holding a syringe for harvesting tissue inserted therein.
Figure 4 is a detailed view of the front portion of the guiding device of figure 3 with the apical portion of the harvesting cannula protruding therefrom.
Figure 5 schematically depicts how to use the guiding device for collecting ADSCs from subcutaneous adipose tissue without harming the patient.
Figures 6 and 7 are perspective views from different points of a guiding device according to another embodiment of the invention, with a side locking tooth for retaining the syringe.
Figure 8 depicts an alternative embodiment of a guiding device of the invention, with two locking teeth for holding a flange of a syringe.
Figure 9 is a picture of a prototype of a plunger lock for a syringe, having a tooth configured to engage with a corresponding side hole of the cylindrical holder to hinder a plunger of a syringe from advancing when the inside of the syringe is under vacuum.
Figures 10a, 10b and 10c show how to install the plunger lock onto the cylindrical holder of the guiding device to make the tooth engage with the side hole.
Figures 11a, 11b, 11c, 11d are different views of the plunger lock of figure 9 when mounted on the guiding device of the invention to stop the plunger of a syringe.
Figures 12 and 13 are a top view and a side view, respectively, of a prototype of a syringe holder of a kit of the invention for storing syringes filled with adipose tissue containing SVF and ADSCs.
Figure 14 is a perspective view of the syringe holder of figures 12 and 13.
Figures from 15 to 25 and from 27 to 39 illustrate a sequence of operations to be performed for using the guiding device and the syringe holder.
Figure 26 depicts a device for harvesting adipose tissue comprising the guiding device of figure 8 and a syringe with a harvesting cannula inserted in the hollow cylindrical holder.

### DETAILED DESCRIPTION

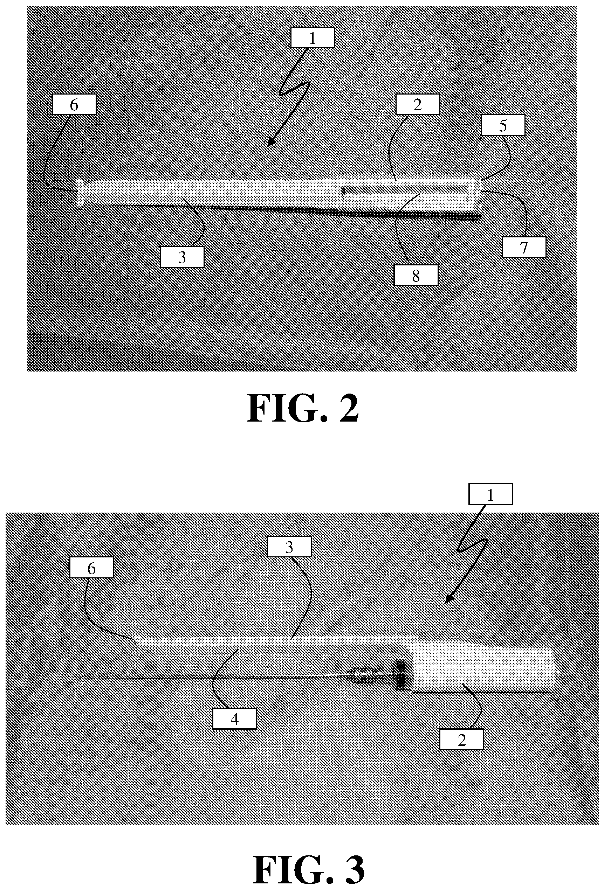

The invention provides a device, an embodiment of which is depicted in figure 2, for guiding insertion and movement of a harvesting cannula below the skin without any risk of harming the patient and at the correct depth in order to collect the adipose tissue with the highest concentration in SVF and ADSCs. The guiding device 1 substantially comprises a hollow cylindrical holder 2 of a syringe for harvesting procedure and a cantilever straight bar 3 integral with the cylindrical holder 2. As shown in figure 3, the cantilever straight bar 3 has a planar abutting surface 4 destined to come into contact with the skin of the patient.

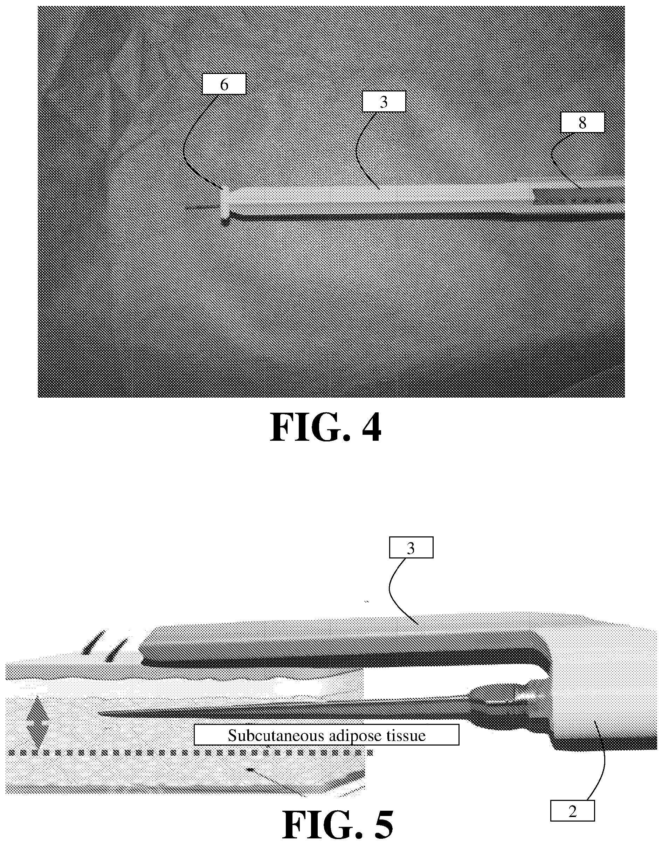

A syringe equipped with a harvesting cannula is inserted in the cylindrical holder 2, so as the barrel of the syringe is held coaxially with the holder 2 and the barrel flange is abutted against an end surface 5 of the holder. According to an aspect, the cylindrical holder 2 is shaped so as to keep the longitudinal axis of the harvesting cannula parallel to the planar abutting surface 4 and at a pre-established distance therefrom. The length of the cantilever straight bar 3 is determined in order to make the apical portion of the harvesting cannula protrude projectively beyond the tip 6 of the guiding device 1 exactly for said pre-established distance, as it may be better appreciated in figures 4 and 5. This pre-established distance is determined so as to correspond to the depth at which the harvesting cannula should be inserted into the patient's skin to reach the subcutaneous adipose tissue.

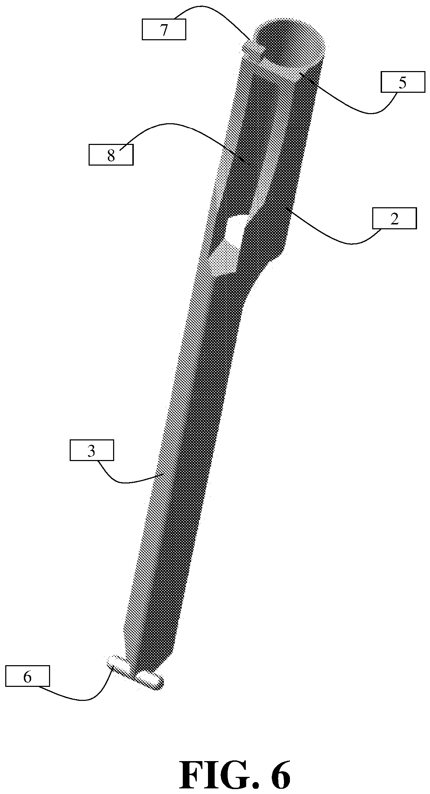

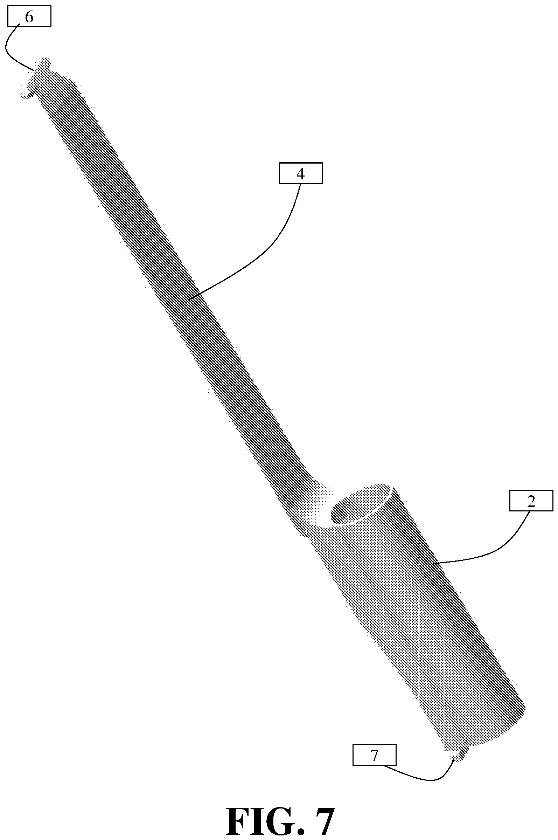

According to an aspect, the guiding device 1 has a locking tooth 7 integral with the cylindrical holder 2, that may be formed either in correspondence of a plane of symmetry of the cylindrical holder 2, as shown in figure 2, or at a di stance therefrom, as shown in the other embodiment depicted in figures 6 and 7. The locking tooth 7 is shaped so as to lock the barrel flange of the syringe, in order to make easier to pull the plunger backwards to create negative pressure inside the syringe. In practice, the syringe with the harvesting cannula is inserted in the cylindrical holder 2 and the barrel flange is rotated to as to engage the locking tooth 7 and being retained thereby. Clearly, the position of the locking tooth 7 is irrelevant provided that it allows to retain the barrel flange. In this configuration, an user may pull backwards the plunger of the syringe with the barrel firmly held by the guiding device 1.

Figure 5 provides a pictorial explanation of how to use a guiding device of the invention. First, the end portion of the harvesting cannula is moved to penetrate throughout the skin as far as the tip 6 of the cantilever straight bar 3 comes into contact with the skin. When this happens, the cannula has attained the subcutaneous adipose tissue, and thus the syringe is rotated of 90 degrees so as to abut the planar surface 4 of the straight bar 3 against the abdomen skin. In this condition, the guiding device 1 is moved back and forth to collect subcutaneous adipose tissue whilst the planar abutting surface 4 of the cantilever straight bar 3 slides in contact with the skin of the abdomen of the patient.

With the guiding device 1, the insertion of the cannula is driven exactly at the correct depth for being immersed in the subcutaneous adipose tissue, which may be for example an adipose layer of the abdomen, and its motion is guided so as to remain immersed therein without any risk of wounding inner organs of the patient
and to harvest the tissue in the proper layer. It may be appreciated that insertion of the harvesting cannula and collection of adipose tissue is outstandingly simplified and is intrinsically safe, thus it may be carried out also by less qualified persons than the plastic surgeon.

According to an aspect, in order to prevent the patient from being scratched by sharp edges of the free end of the cantilever straight bar 3, a tip 6 with rounded edges is preferably formed integral with the cantilever straight bar 3. In the embodiments of figures 4 and 7 the rounded tip 6 is substantially cylindrical, but any other shape free from sharp edges may be imparted.

According to an aspect, the hollow cylindrical holder 2 has a side opening 8 that leaves uncovered the barrel of the syringe, to make the user see how much adipose tissue is being aspired. According to an aspect, the side opening 8 is longitudinally aligned with the cantilever straight bar 3.

The guiding device may be made of plastic or of any material suitable to be used in contact with the patient's body during surgical operations.

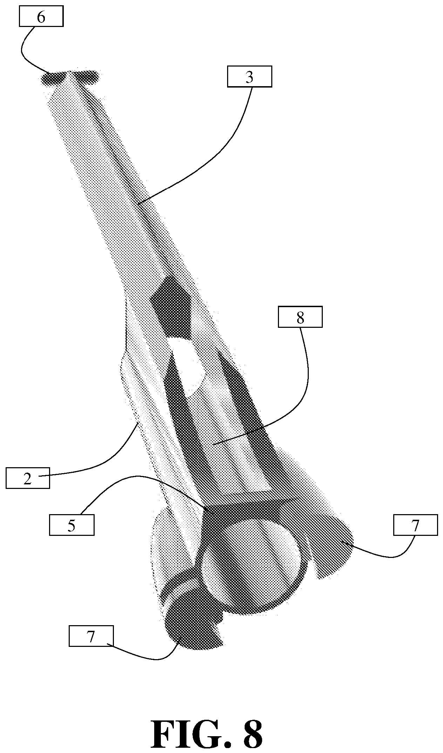

According to an alternative embodiment, depicted in figure 8, a guiding device of the invention may be equipped with two locking teeth 7 for locking a barrel flange of a syringe.

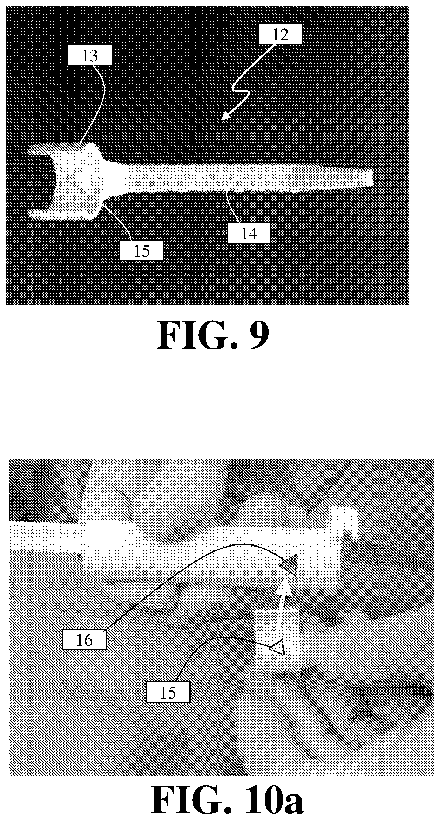

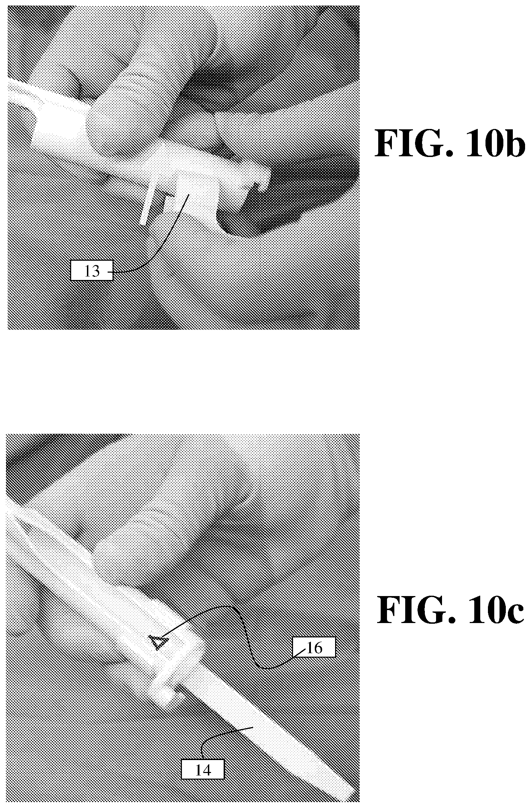

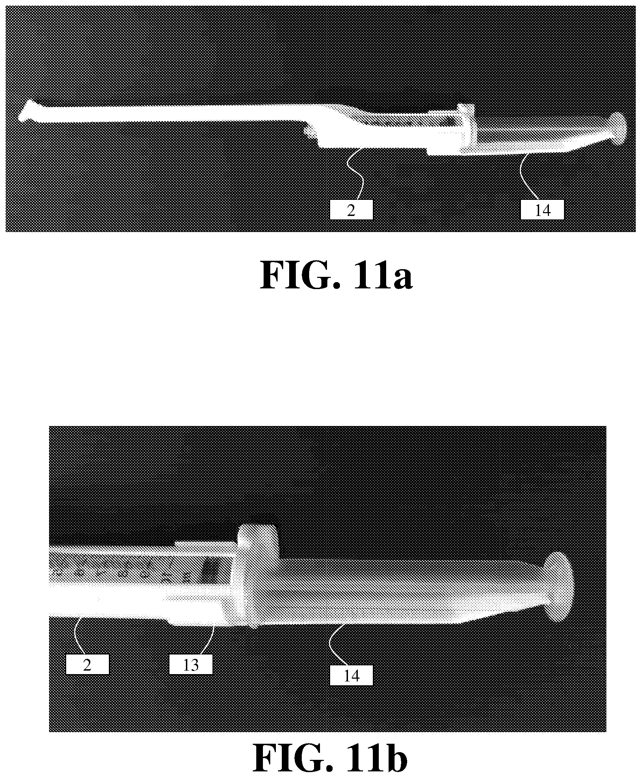

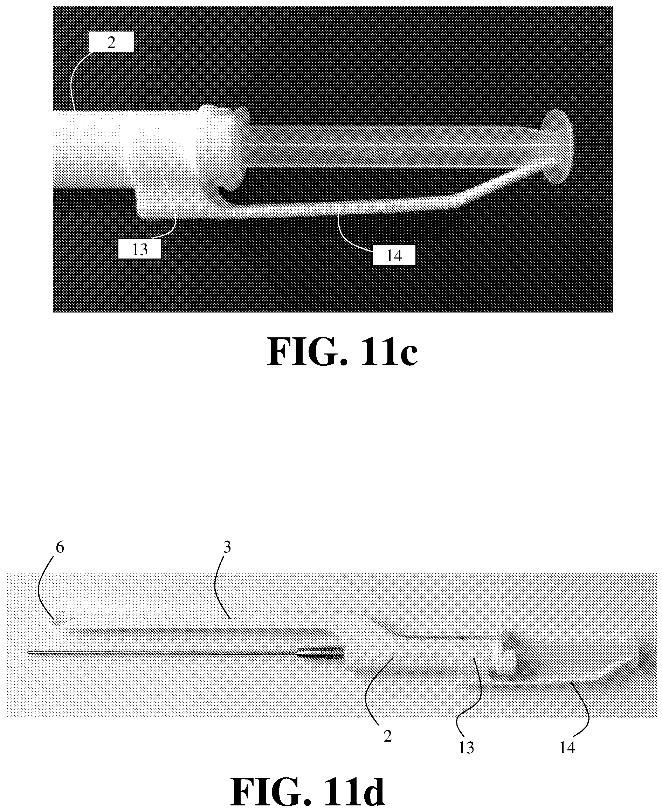

According to an aspect, the guiding device of the invention may be equipped with a plunger lock for a syringe of the type shown in figure 9. The plunger lock 12 has an elastically deformable transversal arched portion 13 configured to embrace at least partially the hollow cylindrical holder 2 so as to grasp it, and a longitudinal stem 14 integral with the arched portion 13. According to an aspect, on the internal surface of the arched portion 13 there may be a tooth 15 that protrudes inward. As shown in the sequence of pictures of figures 10a to 10c, on the cylindrical holder 2 of the guiding device there is a hole or a recess 16 on the outer surface of the holder 2 that matches with the tooth 15 so as when the tooth 15 engages with the hole or recess 16 (figure 10c), the plunger lock 12 is hindered from sliding onto the outer surface of the holder 2. When the arched portion is mounted around the hollow cylindrical holder 2, as shown in the different views of figures 11a to 11c, the stem 14 lays longitudinally and hinders the plunger of the syringe to advance when the syringe is under vacuum. Therefore, it is possible to keep the inside of the syringe under vacuum without using the fingers to keep the plunger in a retracted configuration.

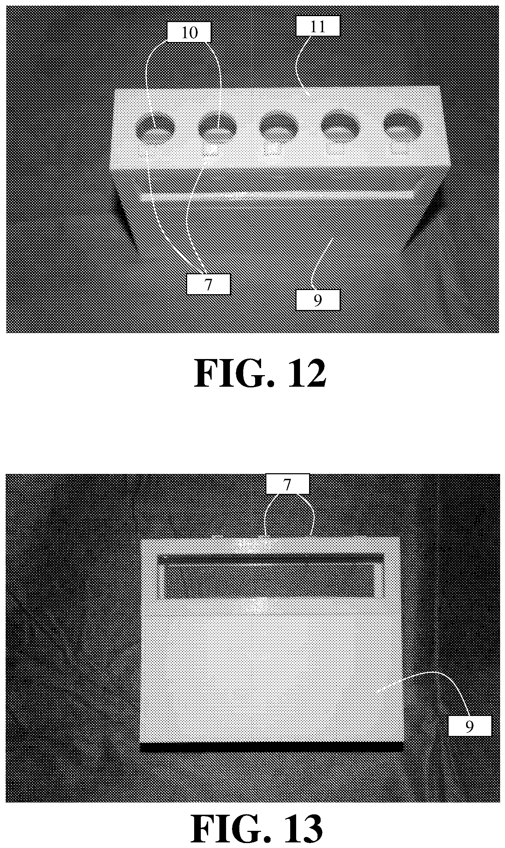

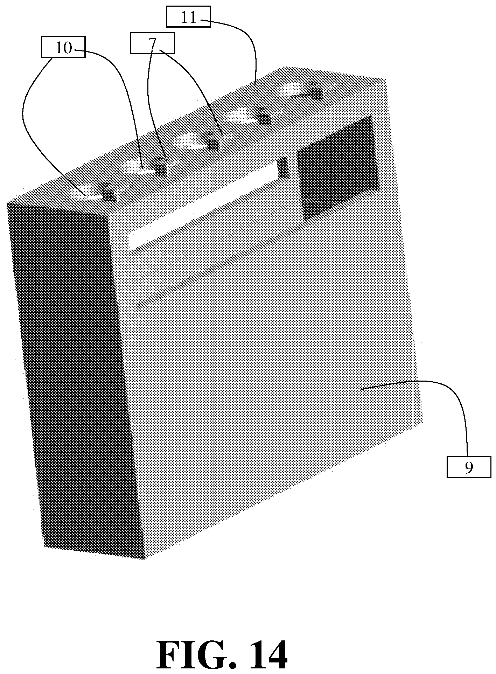

A syringe holder 9 is also provided, of the type depicted in figures 12 to 14. The syringe holder 9 may be realised as a hollow box with circular openings 10 on a top surface 11, wherein the circular openings 10 are shaped so as to let a barrel of a syringe enter therein and the barrel flange not pass therethrough. The syringes are hung with their barrel flanges abutted against the top surface 11 and the barrels protected into the hollow box pre-filled with cold Ringer lactate. Preferably, locking teeth 7 are realised on the top surface 11 and integral therewith, close to the circular openings 10 to lock the barrel flanges of syringes as in the cylindrical holder 2.

In practice, once the syringe is filled of subcutaneous adipose tissue up to about half the syringe capacity, the harvesting cannula is removed and the syringe is filled of Ringer lactate in order to washout the anaesthetic solution. Then a cover cap is put on the syringe adaptor and the syringe is placed at rest for a certain time in the hollow box of figure 8, with the syringe adaptor oriented toward the bottom. After a suitable amount of time, the tissue containing stem cells are separated and washing liquid to be discarded is accumulated in correspondence of the tip of the syringe, and thus it can be easily pushed out of the syringe.

The syringe holder 9 may be sold together with the guiding device 1 as a sterile kit for taking adipose derived stem cells from a subcutaneous adipose tissue below the skin of the patient.

The use of a guiding device and of a sterile kit of the invention is illustrated in the sequence of pictures from 15 to 25 and from 27 to 39.

The procedure is to be performed in sterile conditions: the skin should be carefully cleaned with a disinfecting solution both in the harvesting area and in the implantation area prior to the procedure.

The area where the tissue is harvested should be chosen based on the following criteria:
- adequate quantity of subcutaneous adipose tissue;
- size of the area: the harvesting area should be at least 20 cm in diameter;
- the most common harvesting areas are: the abdomen, the hips and the trochanteric region.

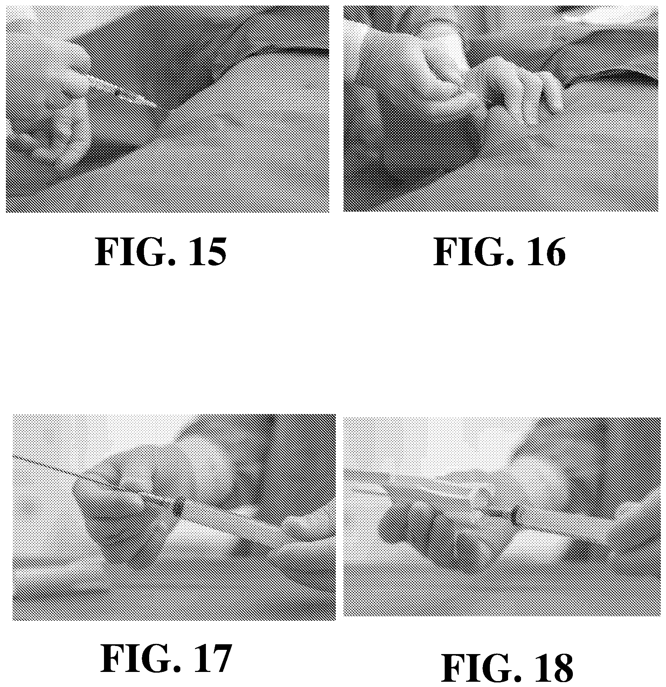

Using local anaesthetic containing adrenalin is recommended. Choose the spot for insertion of the cannula: it should be 1 cm out of the perimeter of the harvesting area (about 20 cm in diameter). Use the 27G needle to inject (Figure 15) the anaesthetic solution.

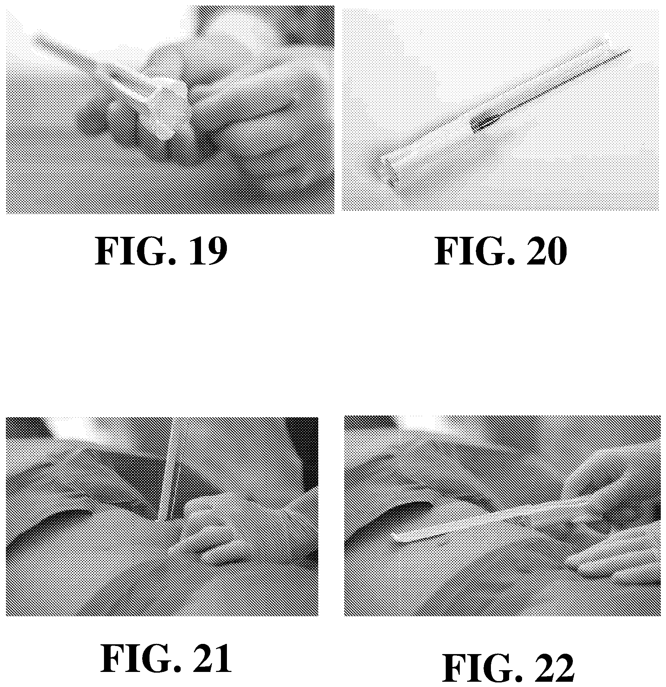

Make an opening in the skin (Figure 16) with the 18G needle. Connect the multi-hole harvesting cannula with the luer lock syringe (Figure 17) containing local anaesthetic. Insert the syringe with the cannula (Figure 18) into the holder 2, then rotate the syringe (Figure 19) so that the barrel flange is engaged in the locking teeth (or tooth) 7 defined on the base abutting surface 5 of the barrel flange. Make sure the cannula is parallel to the cantilever straight bar 3 (Figure 20); if not so, set it in position with a delicate manual move.

Between thumb and forefinger of the non-dominant hand, pinch the skin (Figure 21) where the opening has been previously made with the 18G needle and insert the tip of the cannula, then rotate the cannula by 90 degrees (Figure 22) so as to set the external guide parallel to the skin plane.

Infiltrate the local anaesthesia with a back and forth movement of the cannula while maintaining the straight bar 3 parallel and with the planar abutting surface 4 adhering to the skin.

The anaesthetic solution should be injected in the whole harvesting area. If more anaesthetic is necessary, repeat the same procedure after recharging the syringe with more anaesthetic solution, and wait about 15 minutes.

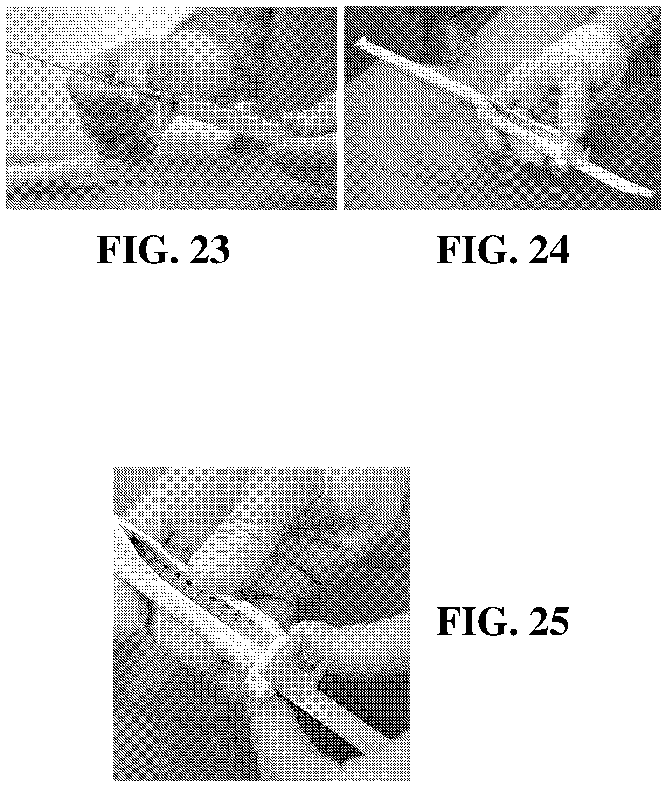

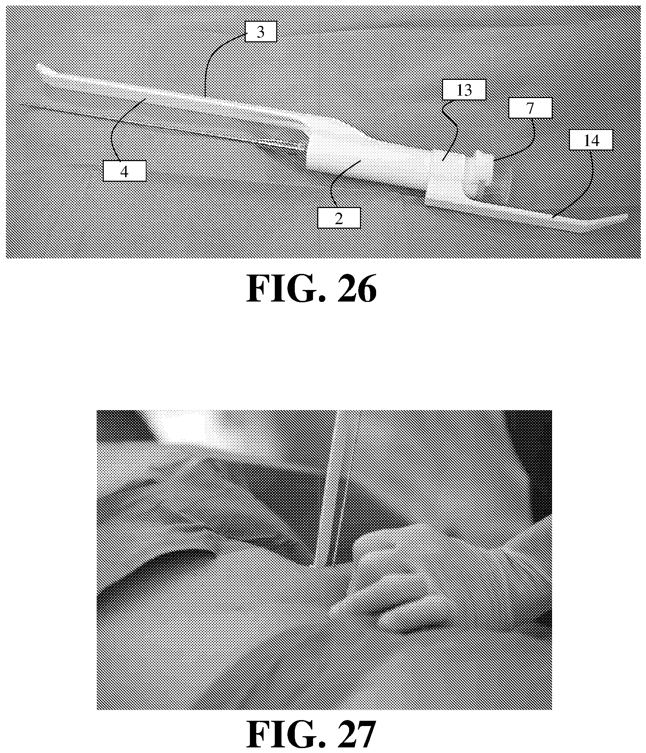

Connect the multi-hole harvesting cannula (Figure 23) with the luer lock syringe, connect the plunger lock to the handpiece (figures 10a, 10b), insert the syringe with the cannula (Figure 24) into the cylindrical holder 2 and rotate the syringe (Figure 25) so that the barrel flange is engaged in the locking teeth (or tooth) 7. Make sure the cannula is parallel to the cantilever straight bar 3; if not so, set it in position with a delicate manual move. The device for harvesting subcutaneous adipose tissue, comprising a guiding device and a syringe with a harvesting cannula inserted in the cylindrical holder 2, will appear as shown in figures 11d and 26.

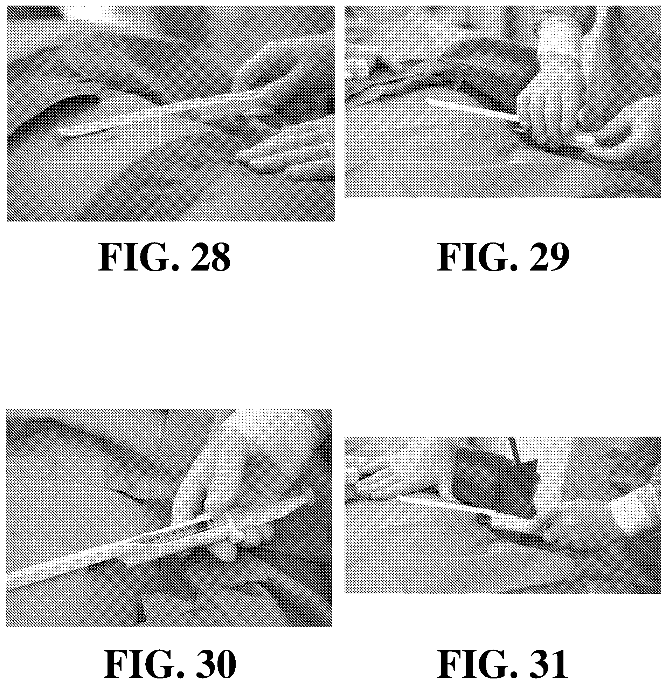

Between thumb and forefinger of the non-dominant hand, pinch the skin (Figure 27) where the opening has been previously made with the 18G needle (figure 16), and insert the tip of the cannula into the skin opening perpendicularly until the tip 6 of the cantilever straight bar 3 touches the skin. Rotate the cannula by 90 degrees (Figure 28) so as to set the cantilever straight bar 3 parallel to the skin plane and the cannula in a subcutaneous plane. While holding the device with the dominant hand (Figure 29), pull the syringe plunger back so as to create the vacuum in the syringe barrel. The plunger lock system, of the type depicted in Figure 9, will hold the plunger (Figure 30) so as there is vacuum insider the barrel of the syringe without the need to maintain the plunger with the dominant hand.

While maintaining the cantilever straight bar 3 parallel and with the planar abutting surface adhering to the skin (Figure 31), move the cannula back and forth in the whole harvesting area for sucking tissue into the barrel. The amount of harvested tissue can be checked through the side opening 8 in the hollow cylindrical holder 2, as schematically indicated by the arrow. Stop as soon as about half the syringe capacity of tissue has been collected in the syringe.

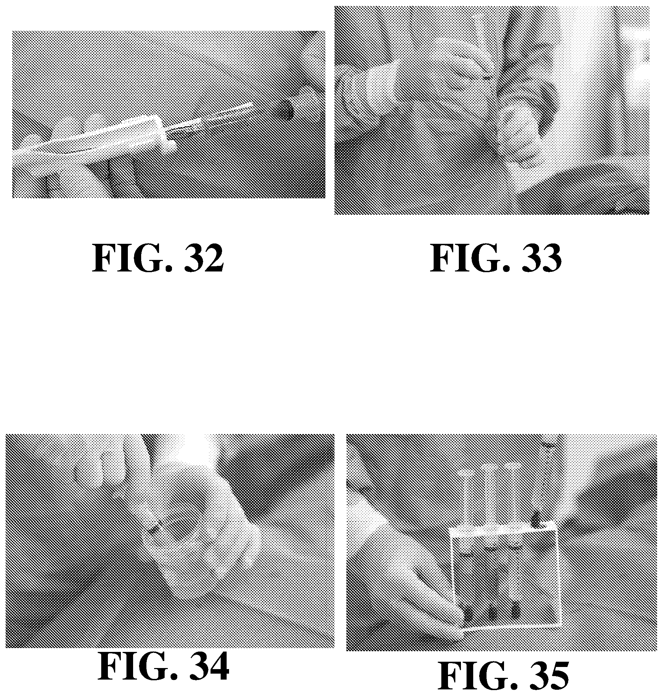

Pull out the cannula from the skin and rotate the syringe until the plunger flange is released from the locking teeth (or tooth) 7 in the back of the holder 2. Pull out the syringe (Figure 32) from the guide and disconnect the cannula (Figure 33). Fill the syringe with sterile Ringer Lactate (Figure 34) previously poured into a container and use a luer lock plug to close the syringe. Put the syringe (plug down) in the syringe holder 9 of the sterile kit (Figure 35). After few minutes, the syringes in the syringe holder 9 will show a separation by gravity of the tissue (top) from the Ringer lactate solution (bottom).

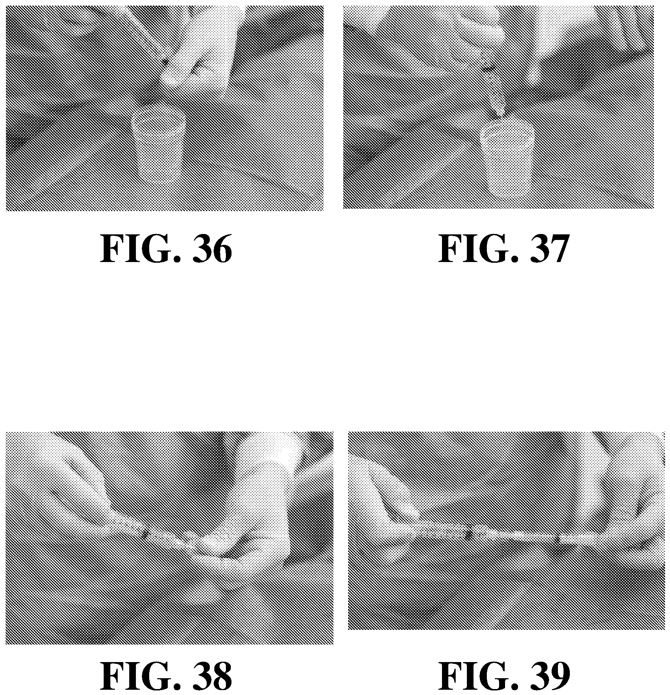

Pull out the syringe from the syringe holder 9, hold it in a vertical position without tilting it or turning it upside down, remove the plug (Figure 36) and discard the washing Ringer Lactate solution (Figure 37) while retaining all the tissue inside the syringe. At the end of this procedure a fat tissue with SVF and ADSCs is obtained; this tissue is already enough fluid to be injected.

According with the Regenerative therapy purposes, the tissue could be concentrated in term of cellularity through a gently centrifugation, or increase the fluidity despite of the viability of adipocyte through a procedure of tissue transfer between to syringes. In any case it is suggested to transfer the tissue in smaller syringes.

Pull out the syringe from the stand, remove the plug (Figure 38) and connect the luer lock transfer connector. Connect the syringe at the other end of the transfer connector (Figure 39) and transfer the tissue, which is now ready to be injected.

## Claims

1. A guiding device (1) for inserting and guiding a harvesting cannula below the skin of a patient, comprising:
a hollow cylindrical holder (2);
a cantilever straight bar (3), integral with the cylindrical holder (2), having a planar abutting surface (4) adapted to slide in contact with the skin of the patient when the harvesting cannula is immersed in a subcutaneous adipose tissue below the skin of the patient;
said hollow cylindrical holder (2) being shaped so as to hold a longitudinal axis of said harvesting cannula parallel to said planar abutting surface (4) and at a pre-established distance from said planar abutting surface (4) equal to a depth at which there is said subcutaneous adipose tissue below the skin;
said cantilever straight bar (3) having a longitudinal length such that to make a tip (6) of the harvesting cannula protrude beyond a distal end of the cantilever straight bar (3) exactly for a length corresponding to said pre-established distance **characterized in that** it further comprises a syringe equipped with the harvesting cannula, a barrel and a plunger sliding inside the barrel and wherein barrel is mounted in said hollow cylindrical holder (2) in a removable manner;
wherein said hollow cylindrical holder (2) has a side opening (8) configured to let a usert see the barrel of the syringe housed into said hollow cylindrical holder (2) whilst it is filled with cells of said subcutaneous adipose tissue.

2. The guiding device (1) of claim 1, wherein said cantilever straight bar (3) terminates with a rounded tip (6) in correspondence of said free end.

3. The guiding device (1) of claim 1, wherein said hollow cylindrical holder (2) has a base abutting surface (5) of a barrel flange of said syringe, and a locking tooth (7) integral with the hollow cylindrical holder (2) for retaining the barrel flange whilst the plunger of the syringe is pulled backwards.

4. A sterile kit for harvesting adipose derived stem cells from a subcutaneous adipose tissue below the skin of a patient, comprising:
a guiding device (1) according to one of claims from 1 to 3,
a syringe holder (9) in the form of a hollow box having a top surface (11)wherein circular openings (10) are obtained, said circular openings (10) being shaped so as to let said barrel of a syringe enter therein and to retain a barrel flange to not pass therethrough, said syringe holder being configured to keep the harvesting syringes hung and their barrels protected into said hollow box, **characterized in that** said hollow box (9) comprises inspection lateral openings of said hung harvesting syringes.

5. The sterile kit of claim 4, wherein said syringe holder (9) comprises locking teeth (7) engageable by said barrel flanges, each locking tooth of said locking teeth (7) being integral with said top surface (11) and being formed at a perimeter of a corresponding circular opening of said circular openings (10).

6. The sterile kit of claim 4, wherein said circular openings (10) are placed on said top surface (11) in a linear succession.

## Patentansprüche

1. Führungsvorrichtung (1) zum Einführen und Führen einer Entnahmekanüle unter der Haut eines Patienten, umfassend:
einen hohlen zylindrischen Halter (2);
einen freitragenden geraden Stab (3), der mit dem zylindrischen Halter (2) einstückig ausgebildet ist, aufweisend eine ebene Anlagefläche (4), die dazu geeignet ist, in Kontakt mit der Haut des Patienten zu gleiten, wenn die Entnahmekanüle in einem subkutanen Fettgewebe unter der Haut des Patienten eingetaucht ist;
wobei der genannte hohle zylindrische Halter (2) so geformt ist, um eine Längsachse der genannten Entnahmekanüle parallel zur genannten ebenen Anlagefläche (4) und in einem vorherbestimmten Abstand von der genannten ebenen Anlagefläche (4) zu halten, der einer Tiefe gleich ist, bei der das genannte subkutane Fettgewebe unter der Haut vorliegt;
wobei der genannte freitragende gerade Stab (3) eine längslaufende Länge derart hat, dass eine Spitze (6) der Entnahmekanüle über ein distales Ende des freitragenden geraden Stabs (3) genau um eine Länge hinausragt, die dem genannten vorherbestimmten Abstand entspricht, **dadurch gekennzeichnet, dass** sie ferner eine Spritze umfasst, die mit der Entnahmekanüle, einem Zylinder und einem Stößel ausgestattet ist, der innerhalb des Zylinders gleitet und worin der genannte Zylinder im genannten hohlen zylindrischen Halter (2) in einer abnehmbaren Weise montiert ist;
worin der genannte hohle zylindrische Halter (2) eine seitliche Öffnung (8) hat, die dazu eingerichtet ist, zu bewirken, dass ein Benutzer den Zylinder der Spritze sieht, der im genannten hohlen zylindrischen Halter (2) beherbergt ist, während er mit Zellen des genannten subkutanen Fettgewebes gefüllt ist.

2. Führungsvorrichtung (1) nach Anspruch 1, worin der genannte freitragende gerade Stab (3) mit einer gerundeten Spitze (6) an dem genannten freien Ende endet.

3. Führungsvorrichtung (1) nach Anspruch 1, worin der genannte hohle zylindrische Halter (2) eine Grund-Anlagefläche (5) eines Zylinderflansches der genannten Spritze und einen Sperrzahn (7) hat, der mit dem hohlen zylindrischen Halter (2) einstückig ausgebildet ist, um den Zylinderflansch zurückzuhalten, während der Stößel der Spritze rückwärts gezogen ist.

4. Steriles Kit zur Entnahme von Stammzellen aus Fettgewebe von einem subkutanen Fettgewebe unter der Haut eines Patienten, umfassend:
eine Führungsvorrichtung (1) nach einem der Ansprüche 1 bis 3,
einen Spritzenhalter (9) in Form eines hohlen Kastens mit einer oberen Fläche (11), in welcher kreisförmige Öffnungen (10) ausgebildet sind, wobei die genannten kreisförmigen Öffnungen (10) so geformt sind, dass sie den genannten Zylinder einer Spritze darin eintreten lassen und einen Zylinderflansch zurückhalten, damit er nicht dadurch tritt,
wobei der genannte Spritzenhalter dazu eingerichtet ist, die Entnahmespritzen gehängt und ihre Zylinder im genannten hohlen Kasten geschützt zu halten, **dadurch gekennzeichnet, dass** der genannte hohle Kasten (9) seitliche Inspektionsöffnungen der genannten gehängten Entnahmespritzen umfasst.

5. Steriles Kit nach Anspruch 4, worin der genannte Spritzenhalter (9) Sperrzähne (7) umfasst, die von den genannten Zylinderflanschen in Eingriff bringbar ist, wobei jeder Sperrzahn der genannten Sperrzähne (7) mit der genannten oberen Fläche (11) einstückig ausgebildet und an einem Umfang einer entsprechenden kreisförmigen Öffnung der genannten kreisförmigen Öffnungen (10) geformt ist.

6. Steriles Kit nach Anspruch 4, worin die genannten kreisförmigen Öffnungen (10) an der genannten oberen Fläche (11) in einer linearen Aufeinanderfolge angeordnet sind.

## Revendications

1. Dispositif de guidage (1) destiné à insérer et à guider une canule de collecte sous la peau d'un patient, comprenant :
un support cylindrique creux (2) ;
une barre droite en porte-à-faux (3), solidaire du support cylindrique (2), ayant une surface de butée plane (4) adaptée pour coulisser en contact avec la peau du patient lorsque la canule de collecte est immergée dans un tissu adipeux sous-cutané sous la peau du patient ;
ledit support cylindrique creux (2) étant formé de manière à maintenir un axe longitudinal de ladite canule de collecte parallèle à ladite surface de butée plane (4) et à une distance préétablie de ladite surface de butée plane (4) égale à une profondeur à laquelle se trouve ledit tissu adipeux sous-cutané sous la peau ;
ladite barre droite en porte-à-faux (3) ayant une longueur longitudinale telle que pour faire saillir une pointe (6) de la canule de collecte au-delà d'une extrémité distale de la barre droite en porte-à-faux (3) exactement sur une longueur correspondant à ladite distance préétablie, **caractérisée en ce qu'**elle comprend en outre une seringue équipée de la canule de collecte, un cylindre et un piston coulissant à l'intérieur du cylindre et dans lequel ledit cylindre est monté dans ledit support cylindrique creux (2) de manière amovible ;
dans lequel ledit support cylindrique creux (2) a une ouverture latérale (8) configurée pour permettre à un utilisateur de voir le cylindre de la seringue logé dans ledit support cylindrique creux (2) pendant qu'il est rempli de cellules dudit tissu adipeux sous-cutané.

2. Dispositif de guidage (1) selon la revendication 1, dans lequel ladite barre droite en porte-à-faux (3) se termine par une pointe arrondie (6) en correspondance avec ladite extrémité libre.

3. Dispositif de guidage (1) selon la revendication 1, dans lequel ledit support cylindrique creux (2) a une surface de butée de base (5) d'une bride de cylindre de ladite seringue, et une dent de verrouillage (7) solidaire du support cylindrique creux (2) pour retenir la bride de cylindre tandis que le piston de la seringue est tiré vers l'arrière.

4. Kit stérile pour collecter des cellules souches dérivées adipeuses à partir d'un tissu adipeux sous-cutané sous la peau d'un patient, comprenant :
un dispositif de guidage (1) selon l'une des revendications de 1 à 3,
un porte-seringue (9) sous la forme d'une boîte creuse ayant une surface supérieure (11) dans laquelle des ouvertures circulaires (10) sont obtenues, lesdites ouvertures circulaires (10) étant formées de manière à permettre audit cylindre d'une seringue d'y entrer et à retenir une bride de cylindre pour ne pas la traverser,
ledit porte-seringue étant configuré pour maintenir les seringues de collecte suspendues et leurs cylindres protégés dans ladite boîte creuse, **caractérisé en ce que** ladite boîte creuse (9) comprend des ouvertures latérales d'inspection desdites seringues de collecte suspendues.

5. Kit stérile selon la revendication 4, dans lequel ledit support de seringue (9) comprend des dents de verrouillage (7) pouvant être engagées par lesdites brides de cylindre, chaque dent de verrouillage desdites dents de verrouillage (7) étant solidaire de ladite surface supérieure (11) et étant formée à un périmètre d'une ouverture circulaire correspondante desdites ouvertures circulaires (10).

6. Kit stérile selon la revendication 4, dans lequel lesdites ouvertures circulaires (10) sont placées sur ladite surface supérieure (11) dans une succession linéaire.
